# EUROPEAN PATENT APPLICATION

(11) **EP 1 114 643 A2**
(43) Date of publication of application: **11.07.2001**
(21) Application number: 00600014.5
(22) Date of filing: 08.12.2000
(51) Int. Cl.: A61K 35/78, A61P 17/00

(54) **Herb containing ointment for the therapy of dermatologic diseases**

(30) Priority: 09.12.1999 GR 99100429
(71) Applicant: Thellesa, Karaj Skender, 115 26 Abelokipi, Athens (GR)
(72) Inventor: Thellesa, Karaj Skender, 115 26 Abelokipi, Athens (GR)

(57) **Abstract**

The pharmaceutical ointment - cream is composed of herbs and chemical substances and is recommended for use to cure various types of eczemas, lupus, psoriasis, hemorrhoids, fungi, and has side effects. The ointment should be kept in a cool place in a glass or porcelain vessel.

The curing period varies from two weeks to 9 months depending on the case.

## Description

The present invention relates to a new ointment, the application of which accomplishes complete cure of various types of eczema (inherited eczema, from stress, caused by chemical substances, etc.), fungi, allergies, trichophyton condylomas, hemorrhoids, acne, lupus, psoriasis.

The recent commercially available pharmaceutical preparations do not provide effective therapy for the above mentioned dermatopathies.

The treatment of eczemas and other dermatological diseases with the ointment of the present invention has given positive results of complete therapy and the diseases did not reappear.

### Synthesis

The ointment is composed of:
a) Plant extract (oil of black cedar)
b) Vaseline
c) Boric acid (H₃BO₃)
d) Solution of HCI
e) SPYRDOUKLI

### Process of Preparation

Vaseline is used as ointment base.
a) Plant extract (oil of black cedar)
b) Vaseline
c) Boric acid (H₃BO₃)
d) Solution of HCI 5% (100 ml H₂O + 2 ml HCL)
e) SPYRDOUKLI

The above ingredients are mixed well until an ointment is formed in a glass vessel or a vessel of porcelain or rubber, but not in a plastic or metallic vessel.

### Forms of Application

The effect of the ointment on the eczema is tranquillizing and stops itching and pain. After a period of time from the application of the ointment to the skin, small drops of water are appearing on the surface of the skin.

The ointment is used three times daily and as often as itching appears. It is spread gently onto the suffering surface with the fingertips. When there are splits or open injuries in the skin, the ointment has to be applied around those open injuries, not in it, in order to avoid irritation. In case of washings the ointment must be applied ten minutes after that to the skin.

The skin surface with eczemas must not be washed more often than 2-3 times weekly, because water is irritating the skin, thus prolonging the period of therapy.

The therapy for eczema, lupus, psoriasis lasts 3-9 months, while for the other dermatologic diseases the therapy lasts from two weeks to three months.

If the patient does not apply correctly the therapy, there might occur irritation. In that case the application of the ointment should be reduced for a short period.

### Example 1

A female patient, 70 years old, suffered from eczema on the legs with swellings, splits and itching which had appeared in the age of 40 after child birth. For years she was using pharmaceutical ointments without results. In 1980 she used the present ointment for nine months and was cured without any remaining traces of eczema. Until today the eczema did not reappear.

### Example 2

A patient of 40 years age suffered from lupus for four years. After using the present ointment, the lupus disappeared within 10 months and did not reappear.

### Example 3

A female patient, 16 years old, suffered from psoriasis in the abdominal region for about two years. After being treated with the present ointment she was totally cured within 6 months.

### Pharmaceutical Preparations Recognized for the therapy of dermatopathies

The recent pharmaceutical preparations which are commercially available, do not provide successful cure for certain dermatological diseases, as are eczemas, lupus, etc.

The preparations of the present invention in the form of a cream do cure with great success:
a) Eczemas (by birth, inherited, caused by various chemical substances, by stress etc.)
b) Fungi
c) Allergies
d) Trichophytia
e) Condylomas
f) Hemorrhoids
g) Acne
h) Lupus
i) Psoriasis

Advantage of the preparations of the present invention is that after completion of therapy the diseases do not reappear.

The synthesis of the cream formulation consists basely of herbs and various chemical substances, as
a) vaseline
b) oil of black cedar
c) boric acid (H3B03)
d) Solution HCI
e) SPYRDOUKLI

### Method of Preparation of the Cream - ointment formulation

The ointment - cream must be prepared and filled up in a glass vessel or in porcelain or rubber and not in a plastic vessel or a metallic vessel.

### How it reacts on the injured skin

The effect of the ointment on the eczema is tranquillizing and stops any pain and itching.

Five minutes after application the skin surface is moistening. The ointment can be used many times during the day, i.e. three times coating of the suffering areas and repeated application whenever itching appears.

The application is done gently with the fingertips. When the eczema has produced splits or cracks in the skin, the ointment must be placed around the open area of the skin, not onto it, in order to avoid erethisma.

The ointment must be applied at least ten minutes after the happening of washings.

Water is irritating the skin with eczemas and shorterns the time of therapy. Therefore the skin should be washed only 2-3 times weekly in such cases.

The time of curing eczema, lupus, psoriasis is 3-9 months, while for the other dermatopathies it is two weeks until three months.

If the patient does not apply correctly the therapy there might occur irritation. In this case the application of the ointment should be reduced for a short period.

## Claims

1. The ointment consisting of Vaseline, plant extract, oil of black cedar, boric acid (H₃BO₃) and solution of HCI and SPYRDOUKLI for use in the therapy of eczemas and other skin diseases.
